# EUROPEAN PATENT SPECIFICATION

(11) **EP 3 822 292 B1**
(45) Date of publication and mention of the grant of the patent: **26.08.2026**
(21) Application number: 19833337.9
(22) Date of filing: 11.07.2019
(51) Int. Cl.: C07K 2/00, C08F 226/02, C08G 69/10, C07K 7/06, C08B 37/08, C08F 8/30, C08L 5/08

(54) **POLYMER COMPOUND AND PROMOTER FOR INTRODUCING COMPOUND INTO CELLS USING SAME**
POLYMERVERBINDUNG UND PROMOTOR ZUR EINFÜHRUNG EINER VERBINDUNG IN ZELLEN UNTER VERWENDUNG DAVON
COMPOSÉ POLYMÈRE ET PROMOTEUR PERMETTANT D'INTRODUIRE UN COMPOSÉ DANS DES CELLULES FAISANT APPEL À CELUI-CI

(30) Priority: 11.07.2018 JP 2018131529
(43) Date of publication of application: 19.05.2021
(73) Proprietor: Josho Gakuen Educational Foundation, Osaka 535-8585 (JP); ADEKA CORPORATION, Arakawa-ku Tokyo 116-8554 (JP)
(72) Inventor: SAKUMA, Shinji, Hirakata-shi, Osaka 573-0101 (JP); UKAWA, Masami, Hirakata-shi, Osaka 573-0101 (JP); TAKAHI, Shunsuke, Tokyo 116-8554 (JP); FUKUSHIMA, Kazunori, Tokyo 116-8554 (JP); MUTO, Mio, Tokyo 116-8554 (JP); MIYATA, Kohei, Tokyo 116-8554 (JP); TASAKI, Akiko, Tokyo 116-8554 (JP); MATSUMOTO, Hotaru, Tokyo 116-8554 (JP)
(74) Representative: Gille Hrabal Partnerschaftsgesellschaft mbB Patentanwälte
(86) International application number: PCT/JP2019/027455
(87) International publication number: WO 2020/013265

(56) References cited:
- EP-A1- 2 828 332
- EP-A2- 2 035 338
- WO-A1-2016/136708
- WO-A2-2011/008545
- CN-A- 101 759 812
- CN-A- 101 759 812
- CN-A- 102 093 489
- CN-A- 102 093 489
- CN-A- 102 120 781
- CN-A- 103 360 531
- JP-A- 2004 261 024
- XIAO B ET AL: "Preparation and characterization of antimicrobial chitosan-N-arginine with different degrees of substitution", CARBOHYDRATE POLYMERS, APPLIED SCIENCE PUBLISHERS , LTD BARKING, GB, vol. 83, no. 1, 1 January 2011 (2011-01-01), pages 144 - 150, XP027353817, ISSN: 0144-8617, [retrieved on 20100724]
- LIU W G ET AL: "A chitosan-arginine conjugate as a novel anticoagulation biomaterial", JOURNAL OF MATERIALS SCIENCE: MATERIALS IN MEDICINE, KLUWER ACADEMIC PUBLISHERS, BO, vol. 15, no. 11, 1 November 2004 (2004-11-01), pages 1199 - 1203, XP019211990, ISSN: 1573-4838, DOI: 10.1007/S10856-004-5672-1
- MOHY ELDIN MOHAMED S. ET AL: "l-Arginine grafted alginate hydrogel beads: A novel pH-sensitive system for specific protein delivery", ARABIAN JOURNAL OF CHEMISTRY, vol. 8, no. 3, 1 May 2015 (2015-05-01), AMSTERDAM, NL, pages 355 - 365, XP055871394, ISSN: 1878-5352, DOI: 10.1016/j.arabjc.2014.01.007
- CHIU JASPER Z.S. ET AL: "Arginine-tagging of polymeric nanoparticles via histidine to improve cellular uptake", EUROPEAN JOURNAL OF PHARMACEUTICS AND BIOPHARMACEUTICS, vol. 89, 1 January 2015 (2015-01-01), NL, pages 48 - 55, XP093020245, ISSN: 0939-6411, DOI: 10.1016/j.ejpb.2014.11.014
- K. MOHRI ET AL.: "Effects of the Chemical Structures of Oligoarginines Conjugated to Biocompatible Polymers as a Mucosal Adjuvant on Antibody Induction in Nasal Cavities", CHEMICAL AND PHARMACEUTICAL BULLETIN, vol. 66, no. 4, 1 April 2018 (2018-04-01), pages 375 - 381, XP055674869
- A. MANSUR ET AL.: "Design and Development of Polysaccharide-Doxorubicin-Peptide Bioconjugates for Dual Synergistic Effects of Integrin-Targeted and Cell -Penetrating Peptides for Cancer Chemotherapy", BIOCONJUGATE CHEMISTRY, vol. 29, 23 May 2018 (2018-05-23), pages 1973 - 2000, XP055674870

## Description

### TECHNICAL FIELD

The present invention relates to a polymer compound and an agent for promoting introduction of compound into cells using the same. According to the present invention, a compound such as a drug can be efficiently introduced into cells.

### BACKGROUND ART

Biopharmaceuticals such as peptides, proteins, antibodies, and nucleic acids are highly specific for target molecules that causes diseases and are considered to be effective in treating the diseases. About half of the new drugs placed on the market in 2015 are biopharmaceuticals and the biopharmaceuticals are expected to increase further in the future. The pharmacokinetic properties of the biopharmaceuticals are low membrane permeability. Due to the high water solubility and the above-mentioned properties by the high molecular weight, many biopharmaceuticals have been developed as highly invasive injections. Therefore, in addition to the pain of patients, there are problems such as medication control by doctors and high production costs, and therefore biopharmaceutical injections are one of the causes of soaring medical costs

On the other hand, the development of absorption-promoting technology that promotes membrane permeation of drugs at local administration sites, transfers them into systemic blood, and efficiently reaches target sites, is one of the major fields of DDS research. In this regard, prodrugs based on lipophilicity improvement or recognition by transporters may be mentioned as typical techniques. However, these prodrugs are mainly low molecular weight organic compounds and have not been successful in biopharmaceuticals. As a classical absorption enhancer, sodium caprate has been used as an additive for ampicillin suppository, but it is extremely limited. Therefore, it is expected to develop DDS technology that can improve the absorbability of biopharmaceuticals, enable medication management by patients such as oral administration or nasal administration, and can reduce medical expenses.

In the study of the infection mechanism of HIV virus, a protein with high cell membrane permeability has been found, and a membrane permeation promoting technique using this protein is being studied. Specifically, a cell-penetrating peptide has been developed based on the primary structure of the HIV virus protein and is being energetically studied as a DDS carrier. The cell-penetrating peptide is a side-chain oligopeptide with about 10 residues rich in basic amino acids such as arginine and lysine, and oligoarginine or penetration is known as a typical one.

The present inventors disclosed polymer compounds and polysaccharide derivatives which can introduce a drug or a water-soluble high molecular weight substance such as a nucleic acid or a protein into cells or mucosa by applying the cell-penetrating peptide technology. (Patent literatures 1 and 2). By using these polymer compounds and polysaccharide derivatives, it has become possible to introduce low membrane permeable compounds into cells and mucosa with high efficiency, without complicated pretreatment.

Chiu Jasper Z.S. et al., European Journal of Pharmaceutics and Biopharmaceutics, vol. 89, 2015, p. 48-55 discloses a poly(ethyl-cyanoacrylate) (PECA) nanoparticle covalently linked to di-arginine-histidine (designated as PECA-RRH). The linking occurs via a histidine anchoring. Guanidino group density is not disclosed in the document. The polymer may be used as a transportation carrier into cells.

### CITATION LIST

### PATENT LITERATURE

[Patent literature 1] WO 2016/136707
[Patent literature 2] WO 2016/136708

### SUMMARY OF INVENTION

### TECHNICAL PROBLEM

In the polymer compounds and polysaccharide derivatives described in Patent literatures 1 and 2, the cell-penetrating peptide having a main length of 8 amino acids or a cell-penetrating peptide having a longer chain length than that, is expensive. Therefore, it was difficult to develop them as pharmaceutical additives unless their costs were reduced.

In addition, since some cytotoxicity is still observed, it has been desired to develop a technology for promoting membrane permeation of a drug, which is safe for cells.

Therefore, the object of the present invention is to provide a technique for promoting membrane permeation of a drug, which is highly versatile and highly safe.

### SOLUTION TO PROBLEM

The present inventors have conducted intensive studies into a technique for promoting membrane permeation of a drug, which is highly safe, as a result, found that the above problem can be solved by a polymer compound containing a basic peptide having a chain length of 3 to 6 amino acids at an end of a side chain

The present invention is based on the above findings.

Accordingly, the present invention relates to:
[1] a polymer compound having a weight average molecular weight of 1000 or more, wherein the polymer compound has, at an end of a side chain, a basic peptide of chain length of 3 to 6 containing three or more arginine, and comprises a guanidino group density of 0.5 to 20mmol/g, originating from arginine, wherein
   the weight average molecular weight is obtained by GPC analysis using an aqueous solvent, and
   wherein the guanidino group density is determined by measuring a hydrogen atom derived from the main chain of the polymer and a hydrogen atom derived from arginine by ¹H-NMR, respectively, and calculating the amount of the guanidino group derived from arginine.
[2] the polymer compound of item [1], wherein a main chain of the polymer compound is one or a combination of two or more selected from the group consisting of a hydrophilic polymer, an anionic polymer, and a polysaccharide derivative,
[3] the polymer compound of item [1] or [2], wherein an end of the basic peptide is - CONH₂ group
[4] the polymer compound of items [1] to [3], wherein the basic peptide is consisting of arginine and glycine,
[5] an agent for promoting introduction of compound into cells, comprising the polymer compound of items [1] to [4], and
[6] a pharmaceutical composition, comprising the polymer compound of items [1] to [4], and a drug,

The present specification discloses (subject-matter not claimed):
[7] a method for administrating a drug, comprising the steps of: mixing the polymer compound according to any one of the items [1] to [4] and a drug (in particular, low membrane permeable drug), and administering the mixture to a subject in need thereof, in an amount effective thereof,
[8] a polymer compound according to any one of the items [1] to [4], for using in an administration of the drug (in particular, low membrane permeable drug), and
[9] use of the polymer compound according to any one of the items [1] to [4], for preparing a pharmaceutical composition.

### ADVANTAGEOUS EFFECTS OF INVENTION

According to the polymer compound of the present invention, the basic peptide in the side chain has a chain length of 3 to 6 amino acids, and the effect of the present invention can be obtained, for example, even with three arginines. Therefore, compared with conventional long-chain cell-penetrating peptides such as octaarginine, an agent for promoting introduction of compound can be prepared at a low cost in the present invention. In addition, the polymer compound of the present invention can efficiently introduce compounds such as drugs into cells and has low cytotoxicity, and thus, the drugs can be safely introduced.

### DESCRIPTION OF EMBODIMENTS

### [1] Polymer compound

The polymer compound of the present invention has a weight average molecular weight of 1000 or more. The polymer compound has, at an end of a side chain, a basic peptide of chain length of 3 to 6 containing three or more arginine, and comprises a guanidino group density of 0.5 to 20mmol/g, originating from arginine.

### «Molecular weight»

The weight average molecular weight of the polymer compound (in the following also designated as molecular weight) is not limited as long as the effect of the present invention can be achieved. However, the lower limit of the molecular weight is, 1,000 or more, preferably 5,000 or more, more preferably 10,000 or more, more preferably 50,000 or more, more preferably 100,000 or more, more preferably 200,000 or more, more preferably 300,000 or more. The upper limit of the molecular weight is, for example, 50,000,000 or less, preferably 40,000,000 or less, more preferably 30,000,000 or less, more preferably 20,000,000 or less, even more preferably 10,000,000 or less. The lower limit and the upper limit of the molecular weight can be arbitrarily combined, and the invention can be conducted in the range of the combination. If the molecular weight of the polymer compound is too small or too large, the effect of the present invention may not be sufficiently obtained.

The term "molecular weight" used herein means a weight average molecular weight. The "weight average molecular weight" is obtained by GPC analysis using an aqueous solvent, and specifically, it means the weight average molecular converted to pullulan, polyethylene glycol (PEG), or polyethylene oxide (PEO).

### «Basic peptide»

The polymer compound of the present invention has a basic peptide of chain length of 3 to 6 containing three or more arginine at an end of a side chain thereof. The polymer compound of the present invention can efficiently introduce a compound such as a drug into cells by having the basic peptide.

The basic peptide chain length of 3 to 6 3 or more arginine. The upper limit thereof is 6 or less, preferably 5 or less, more preferably 4 or less. Three or more arginines comprised in the basic peptide may be contained in the basic peptide consecutively or discontinuously. The lower limit and the upper limit of arginines contained in the basic peptide can be arbitrarily combined as long as the effect of the present invention can be achieved, and the invention can be conducted in the range of the combination.

The chain length of 3 to 6 is not limited as long as it is 3 to 6. However, it is 3 to 6 in one embodiment, 3 to 5 in one embodiment, 3 to 4 in one embodiment, 3 to 5 in one embodiment, 3 to 4 in one embodiment, 4 to 6 in one embodiment, 4 to 5 in one embodiment. In the basic peptide, all amino acids may be arginine, or it may contain one or more amino acid(s) such as glycine other than arginine.

The basic peptide may contain the amino acid(s) other than arginine as long as it is basic as a whole. As the amino acids, there may be mentioned, for example, basic amino acids (such as ornithine, lysine, hydroxylysine or histidine), neutral amino acids (such as alanine, asparagine, cysteine, glutamine, glycine, isoleucine, leucine, methionine, phenylalanine, proline, serine, threonine, tryptophan, tyrosine, or valine), acidic amino acids (such as aspartic acid, or glutamic acid), but it is preferably basic amino acids or neutral amino acids, more preferably basic amino acids. The number of amino acids other than arginine is preferably 5 or less, more preferably 4 or less, further preferably 3 or less, further preferably 2 or less, further preferably 1 or less, and most preferably 0.

The amino acid contained in the basic peptide may be either L-form amino acid or D-form amino acid, and can be appropriately selected depending on the cell and the water-soluble high molecular weight substance to be introduced. In addition, the amino acid used herein means α-amino acid unless otherwise specified

The basic peptide of chain length of 3 to 6 includes a peptide of RRR (R represents arginine. The same applies hereinafter), RRRR, RRRRR, or RRRRRR, 4-chain length basic peptide with 3 arginine and 1 glycine, 5-chain-length basic peptide with 4 arginine and 1 glycine, 5-chain length basic peptide with 3 arginine and 2 glycine, 6-chain length basic peptide with 5 arginine and 1 glycine, 6-chain long basic peptide with 4 arginine and 2 glycine, or 6-chain long peptide with 3 arginine and 3 glycine. An arrangement of glycine and arginine is not limited, but it is preferable that arginine is located at the end of the side chain.

The basic peptide can be represented by the following general formula (1): 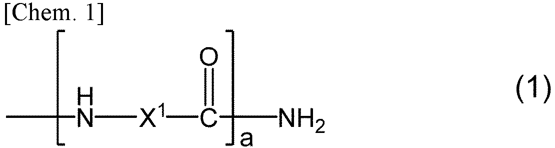 wherein X¹ represents a residue in which the terminal amino group and terminal carboxyl group are removed from arginine, basic amino acid, neutral amino acid, or acidic amino acid, and at least three of X¹ is arginine residue, and a is an integer of 3 to 6, or by the following general formula (2): 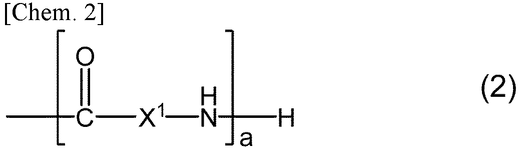 wherein X¹ represents a residue in which the terminal amino group and terminal carboxyl group are removed from arginine, basic amino acid, neutral amino acid, or acidic amino acid, and at least three of X¹ is arginine residue, and a is an integer of 3 to 6.

The terminal of the basic peptide represented by the above formula (1) preferably is -CONH₂ group. The terminal of the basic peptide may be -COOH (carboxyl group). However, if the terminal is -CONH₂ group (or NH₂ group), the dispersibility of the obtained agent for promoting introduction of compound into cells may be improved or the dispersibility of the pharmaceutical composition may be improved.

### «Main chain»

The main chain of the polymer compound used herein means a portion excluding the side chain containing a basic peptide of chain length of 3 to 6 containing three or more arginine.

Specifically, the main chain may be a single main chain or a graft-polymerized main chain. The main chain generally means the longest carbon chain in a compound, and may be partially substituted with heteroatoms. The main chain may have a ring structure and the longest backbone is the main chain.

The polymer compound of the present invention can be produced, for example, by binding the side chain(s) having a basic peptide of chain length of 3 to 6 containing three or more arginine, to the single-chain polymer compound or a graft polymer (hereinafter, referred to as a main chain polymer).

The main chain polymer is not particularly limited. It includes a hydrophilic polymer, an anionic polymer, or a polysaccharide derivative, but a hydrophilic polymer or a polysaccharide derivative is preferable. The hydrophilic polymer used herein means a water-soluble polymer or a polymer that swells in water. The water-soluble polymer can be uniformly dissolved in water, in an amount of 0.1% by mass or more, at 25°C under normal pressure.

The hydrophilic polymer is not particularly limited, but there may be mentioned, for example, polysaccharides such as guar gum, agarose, mannan, glucomannan, polydextrose, lignin, chitin, chitosan, carrageenan, pullulan, chondroitin sulfate, cellulose, hemicellulose, methylcellulose, ethylcellulose, hydroxyethylcellulose, hydroxypropylcellulose, starch, cationic starch, and dextrin, or modified polysaccharides; water-soluble proteins or water-soluble polypeptides such as albumin, casein, gelatin, polyglutamic acid (poly-γ-glutamic acid or poly-α-glutamic acid), and polylysine; vinyl-based hydrophilic polymer such as polyacrylic acid, polymethacrylic acid, poly (hydroxyethyl acrylate), polyacrylamide, polymethacrylicamide, polyN-vinylacetamide, polyvinylpyrrolidone, polyvinyl alcohol, poly (2-aminoethylacrylate), poly (2-aminoethylmethacrylate), acrylic acid-acrylamide copolymer, methacrylic acid-acrylamide copolymer, acrylic acid-N-isopropylacrylamide copolymer, methacrylic acid-N-isopropylacrylamide copolymer, acrylic acid-N-vinylacetamide copolymer, methacrylic acid-N-vinylacetamide copolymer, acrylic acid-maleic acid copolymer, methacrylic acid-maleic acid copolymer, acrylic acid-fumaric acid copolymer, methacrylic acid-fumaric acid copolymer, ethylene-maleic acid copolymer, isobutylene-maleic acid copolymer, styrene-maleic acid copolymer, alkyl vinyl ether-maleic acid copolymer, alkyl vinyl ether-fumaric acid copolymer; and water-soluble polyurethane.

The polysaccharide derivative is not particularly limited, but there may be mentioned, for example, carboxymethylated polysaccharide derivatives such as carboxymethylated starch, carboxymethylated cellulose, and carboxymethylatedβglucose, pectin, pectic acid, hyaluronic acid, or alginic acid.

The anionic polymer is a polymer having an anion group in a side chain. The "anion group" in the anionic polymer can be appropriately selected from the anion groups mentioned regarding to the above the amphoteric polymer.

An embodiment of the anion group includes a carboxylic acid group, a carboxy group, a phosphoric acid group, a sulfonic acid group, a nitric acid group, a boronic acid group. The specific examples of the anionic polymer is not particularly limited, but there may be mentioned polyacrylic acid, polymethacrylic acid, polyglutamic acid, carboxylmethylated polyhistidine, hyaluronic acid, alginic acid, or polyaspartic acid.

As the main chain polymer, one or a combination of two or more of the hydrophilic polymer, the anionic polymer, and the polysaccharide derivative can be appropriately used.

The main chain polymer preferably has a carboxyl group or an amino group in order to easily bind a side chain containing arginine or a side chain containing a basic peptide. By having the carboxyl group, it can be easily bonded to an amino group of an amino acid such as arginine. Also, by having an amino group, it can be easily bonded to a carboxyl group of an amino acid such as arginine.

The main chain polymer is not limited, but preferably does not have membrane permeability and a guanidino group. Since the main chain polymer does not have a guanidino group or membrane permeability, the polymer compound of the present invention itself is difficult to be taken up by cells. Thus, it is considered that only a compound such as a drug which loosely coexists by non-covalent bonds is easily taken up by cells.

### «Side chain»

The side chain of the polymer compound of the present invention is not particularly limited as long as it has a basic peptide of chain length of 3 to 6 containing three or more arginine, at an end thereof. That is, the term "side chain" used herein means a chain having the basic peptide of chain length of 3 to 6 containing three or more arginine, at an end thereof. The term "end of the side chain" means a terminal part of a branched chain which branches off from the main chain and does not re-bond to the main chain.

The side chain containing the basic peptide of chain length of 3 to 6 containing three or more arginine, can contain any chain on the main chain side of one arginine or the basic peptide. The any chain is not particularly limited, but, for example, includes a linker peptide.

Amino acids constituting the linker peptide include neutral amino acids or ω-aminoalkanoic acids. Neutral amino acids include, for example, alanine, asparagine, cysteine, glutamine, glycine, isoleucine, leucine, methionine, phenylalanine, proline, serine, threonine, tryptophan, tyrosine, valine, and hydroxyproline, and ω-aminoalkanoic acids include 3-aminopropanoic acid, 4-aminobutanoic acid, 5-aminopentanoic acid, 6-aminohexanoic acid, 7-aminoheptanoic acid, 8-aminooctanoic acid, 9-aminononanoic acid, 10-aminodecanoic acid, and 11-aminoundecanoic acid, nd any combination thereof can be used. The amino acids are preferably glycine, alanine, valine, isoleucine, leucine, serine, threonine, and phenylalanine, more preferably glycine, alanine, and serine, and most preferably glycine, because the introduction efficiency of the compound introduced into the cells is increased.

The chain length of the linker peptide is not particularly limited as long as the effect of the present invention can be achieved, but is, for example, 1 to 30, preferably 1 to 20, more preferably 1 to 10, more preferably 1 to 5.

### «Guanidino group»

The polymer compound of the present invention comprises a guanidino group density of 0.5 to 20mmol/g, originating from arginine, represented by the following formula (3): 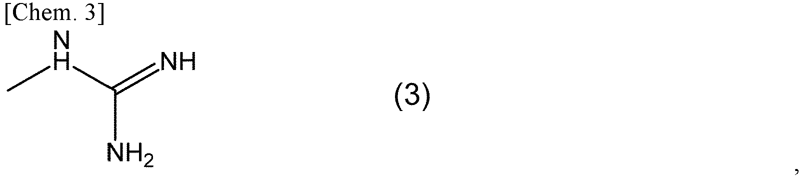 but it is preferably 1.0 to 10.0mmol/g, more preferably 1.0 to 8.0mmol/g, further more preferably 1.5 to 8.0mmol/g. If it is less than 0.5 mmol/g, the effect of the present invention cannot be obtained. If it is more than 20.0 mmol/g, strong toxicity may exhibit depending on the compound to be introduced. When the amount of the guanidino group is in the above range, the compounds such as drug can be efficiently introduced into cells.

The amount of the guanidino group in the polymer compound of the present invention is not limited. For example, ¹H-NMR of the polymer compound is measured to obtain a hydrogen atom derived from the main chain of the polymer and a hydrogen atom derived from arginine, and to calculate the amount of the group derived from arginine. From this result, the density of guanidino groups per polymer compound can be calculated. The specific measurement method is shown in Examples.

### «Method for preparing polymer compound»

A method for preparing the polymer compound of the present invention is not particularly limited. For example, the polymer compound may be prepared by polymerizing polymerizable monomer having a side chain containing a basic peptide of chain length of 3 to 6 containing three or more arginine. Further, the polymer compound may be prepared by introducing the side chain containing a basic peptide of chain length of 3 to 6 containing three or more arginine to the main chain polymer. However, considering the ease of preparation, it is preferably prepared by introducing the side chain containing a basic peptide of chain length of 3 to 6 containing three or more arginine to the main chain polymer.

When the main chain polymer has a carboxyl group, the polymer compound can be prepared by peptide-reacting the carboxyl group with an amino group of the basic peptide. A known method may be used for the reaction between the carboxyl group and the amino group. For example, there may be mentioned a method in which the carboxyl group is succinimide esterified with N-hydroxysuccinimide and then the amino group is reacted.

When the main chain polymer has an amino group, the polymer compound can be prepared by peptide-reacting the amino group with a carboxyl group of the basic peptide. The method for fixing the side chain containing the basic peptide of chain length of 3 to 6 containing three or more arginine, is not limited to the above method, and the side chain can be fixed using generally-known chemical reactions.

When the main chain polymer has a side chain having a carboxyl group or an amino group at the end, the polymer compound of the present invention can be prepared by binding the carboxyl group or the amino group thereof to the basic peptide of chain length of 3 to 6 containing three or more arginine

The polymer compound of the present invention can be used for preparing an agent for promoting introduction of compound or a pharmaceutical composition, described later. Therefore, the use of the present invention is for the preparation of the agent for promoting introduction of compound, or the pharmaceutical composition for treating or preventing a disease.

### [2] Agent for promoting introduction of compound

The agent for promoting introduction of compound of the present invention comprises the polymer compound of the present invention. It can efficiently introduce the compound such as a drug into cells by comprising the polymer compound.

### «Compound to be introduced into cells»

The compound to be introduced into cells by the agent for promoting introduction of compound of the present invention is not particularly limited, but includes proteins (peptides), DNA, RNA, lipids, carbohydrates, or low molecular weight compounds. In particular, the agent for promoting introduction of compound can be used to introduce low membrane permeable compounds with low cell introduction efficiency into cells

The low membrane permeable compound includes, for example, drugs such as a peptide/protein drug such as an insulin, and an insulin secretion promoter (e.g., exendin-4 and GLP-1), a steroid hormone, a non-steroidal analgesic anti-inflammatory drug, a tranquilizer, an anti-hypertensive drug, a therapeutic drug for an ischemic heart disease, an anti-histamine drug, an anti-asthmatic drug, an anti-Parkinson drug, a cerebral circulation improving drug, an anti-emetic drug, an anti-depressant drug, an anti-arrhythmic drug, an anti-coagulant drug, an anti-gout drug, an anti-fungal drug, an anti-dementia drug, a therapeutic drug for Sjögren's syndrome, a narcotic analgesic drug, a beta blocker, a β1 agonist, a β2 agonist, a parasympathomimetic drug, an anti-tumor drug, a diuretic drug, an anti-thrombotic drug, a histamine H1 receptor antagonist, a histamine H2 receptor antagonist, an anti-allergic drug, a smoking cessation drug, and a vitamin; nucleic acid compounds such as a deoxyribonucleic acid (DNA), a ribonucleic acid (RNA), and analogs or derivatives thereof (e.g., a peptide nucleic acid (PNA) and a phosphorothioate DNA); peptide compounds such as an enzyme, an antibody, a glycoprotein, and a transcription factor; and polysaccharide derivatives such as pullulan, amylopectin, amylose, glycogen, cyclodextrin, dextran, hydroxyethyldextran, mannan, cellulose, starch, alginic acid, chitin, chitosan, and hyaluronic acid, and derivatives thereof.

Cells to which the compound introduction promoter of the present invention is applied, may be any cells derived from animals, plants, or bacteria. However, considering the introduction efficiency of the low membrane permeable compound, the cells are preferably derived from mammals such as human, primate simian, canine, feline, ferret, bovine, horse, goat, sheep, guinea pig, hamster, gerbil, mouse or rat. The introduction of the compound into cells may be carried out in vivo or in vitro.

The agent for promoting introduction of compound of the present invention can comprise a diluent, or an additive, in addition to the polymer compound. As the diluent, there may be mentioned purified water, alcohols (e.g., ethanol), distilled water for injection use and physiological saline, propylene glycol, polyethylene glycol, plant oil (e.g., olive oil), or polysorbate 80. Further, as the additive other than the diluent, it may comprise a moistening agent, a suspending agent, a sweetener, a flavor, an antiseptic, an emulsifying agent, a dispersing agent, a stabilizing agent, a solubilizing or solubilization assisting agent.

The agent for promoting introduction of compound of the present invention can be used for the introduction of compound (in particular, low membrane permeable compound) into cells. That is, the use of the present invention is a use of the agent for promoting introduction of compound, for the introduction of compound (in particular, low membrane permeable compound) into cells

### [3] Pharmaceutical composition

The pharmaceutical composition of the present invention comprises a drug and the polymer compound of the present invention. The pharmaceutical composition can efficiently introduce the drug into cells by comprising the polymer compound.

### «Drug»

The drug comprised in the pharmaceutical composition of the present invention is not particularly limited, but includes, for example, drugs such as a peptide/protein drug such as an insulin, and an insulin secretion promoter (e.g., exendin-4 and GLP-1), a steroid hormone, a non-steroidal analgesic anti-inflammatory drug, a tranquilizer, an anti-hypertensive drug, a therapeutic drug for an ischemic heart disease, an anti-histamine drug, an anti-asthmatic drug, an anti-Parkinson drug, a cerebral circulation improving drug, an anti-emetic drug, an anti-depressant drug, an anti-arrhythmic drug, an anti-coagulant drug, an anti-gout drug, an anti-fungal drug, an anti-dementia drug, a therapeutic drug for Sjögren's syndrome, a narcotic analgesic drug, a beta blocker, a β1 agonist, a β2 agonist, a parasympathomimetic drug, an anti-tumor drug, a diuretic drug, an anti-thrombotic drug, a histamine H1 receptor antagonist, a histamine H2 receptor antagonist, an anti-allergic drug, a smoking cessation drug, and a vitamin, and an antibody drug.

The pharmaceutical composition of the present invention can comprise a diluent, or an additive, in addition to the drug and the polymer compound. As the diluent, there may be mentioned purified water, alcohols (e.g., ethanol), distilled water for injection use and physiological saline, propylene glycol, polyethylene glycol, plant oil (e.g., olive oil), or polysorbate 80. Further, as the additive other than the diluent, it may comprise a moistening agent, a suspending agent, a sweetener, a flavor, an antiseptic, an emulsifying agent, a dispersing agent, a stabilizing agent, a solubilizing or solubilization assisting agent.

The dose of the pharmaceutical composition of the present invention is optionally decided by taking into consideration strength of each active ingredient, or symptoms, age, or sex of each patient to be administered. For example, in the case of oral administration, the usual dosage for an adult (60 kg in weight) is 0.1 to 100 mg, preferably 0.1 to 50 mg per day. In the case of parenteral administration, the usual dosage is 0.01 to 50 mg, preferably 0.01 to 10 mg per day in the form of an injection.

### «Method for administrating drug»

The method for administrating the drug of the present invention comprises the steps of: mixing the polymer compound and a drug (in particular, low membrane permeable drug), and administering the mixture to a subject in need thereof, in an amount effective thereof. In the method for administrating the drug, the polymer compound may be used without limitation, and the drug to be administrated is also not particularly limited. Further, according to the method for administrating a drug of the present invention, the drug can be efficiently introduced into cells.

### «Polymer compound for administrating drug»

The polymer compound of the present invention is for use in an administration of the drug (in particular, low membrane permeable drug). That is, it is the polymer compound for administrating the drug, and the above polymer compound and the above drug can be used without limitation. The polymer compound of the present invention can efficiently introduce the drug into cells.

### «Use for preparing pharmaceutical composition»

The use of the polymer compound of the present invention is for preparing the pharmaceutical composition. In the preparation of the pharmaceutical composition, the pharmaceutical composition comprising the polymer compound and the drug is prepared. In the preparation of the pharmaceutical composition, the pharmaceutical composition can be prepared according to a known method for preparing a pharmaceutical composition, except that the polymer compound of the present invention is used.

### «Function»

In the polymer compound of the present invention, the mechanism of efficiently introducing the compound such as the drug into cells has not been fully elucidated, but may be presumed to be as follows. However, the present invention is not limited by the following presumption.

The polymer compound of the present invention has the side chain containing the basic peptide of chain length of 3 to 6 containing three or more arginine. Arginine contained in the side chain has a guanidino group, and the polymer compound of the present invention comprises the guanidino group density of 0.5 to 20mmol/g. By having the above range of the guanidino group density, it is considered that when the polymer compound approaches the cell membrane, a macropinocytosis of the cell is induced. The cell tries to take up the polymer compounds containing arginine by macropinocytosis, but it is difficult to take up the polymer compounds that are macromolecules. On the other hand, it is considered that the compounds such as drugs coexisting with the polymer compounds are taken up into cells, because they have a smaller molecular weight than the polymer compounds. That is, it is presumed that the polymer compound of the present invention can induce the macropinocytosis of cells by containing the guanidino group density of 0.5 to 20mmol/g, whereby the coexisting compounds such as the drugs are taken up into the cells.

On the other hand, it is presumed that the main chain polymer of the polymer compound can induce the micropinocytosis as long as it can bind the side chain containing the basic peptide of chain length of 3 to 6 containing three or more arginine, and thus the effect of the present invention can be exhibited. Therefore, in the polymer compound of the present invention, the main chain polymer is not limited to a specific main chain polymer.

### EXAMPLES

The present invention now will be further illustrated by, but is by no means limited to, the following Examples. The term "parts" and "%" in the examples are based on mass, unless otherwise specified.

### «Example 1» (not according to the invention)

In this example, acrylic acid-N-vinylacetamide copolymer was used as a main chain polymer, to prepare a polymer compound having one arginine in side chains.

### (Synthesis of succinimide of acrylic acid-N-vinylacetamide copolymer)

A copolymer (NVA-AANa polymer) was synthesized by a conventional method using sodium acrylate (30.0g) and N-vinylacetamide (70.0g) as raw materials, with reference to Example 14 of JPA08-081428.

Subsequently, a column tube was filled with a cation exchange resin (IR120B, Organo Corporation), and a 5.0% by weight of NVA-AANa polymer aqueous solution (130.0g) was passed at 2.6 mL/min to obtain an NVA-AA polymer aqueous solution. The obtained NVA-AA polymer aqueous solution was freeze-dried to obtain 5.7 g of NVA-AA polymer.

NVA-AA polymer (5.0g) and DMF (142.0g) were placed in a 300 mL five-necked flask and cooled to 10°C or lower in an ice-bath. To this solution, 58.2 g of DMF solution of 0.2g/mL of N-hydroxysuccinimide was added dropwise at 2.0 mL/min. Further, a DMF solution of 0.4 g/mL of N, N'-dicyclohexylcarbodiimide (66.2g) was added dropwise at 1.0 mL/min thereto. The mixture was stirred under an ice bath for 1 hour, then warmed to room temperature and stirred for 24 hours. A filtrate was recovered by suction filtration of the reaction solution, and reprecipitated with 2 L of acetonitrile. Then, the precipitate was washed with 2 L of acetone and a solid was recovered by suction filtration. The resulting solid was dried under reduced pressure to obtain 5.4 g of a succinimide-esterified NVA-AA polymer (NVA-AA OSu polymer).

### (Introduction of basic peptide)

10.0mg of NVA-AA OSu polymer was dissolved in 1.0mL of DMF. 0.2 mL of DMF solution of 77.5 mg/mL of mono-L-arginine (NH2-R1/L, Merck Corporation) and 0.02 mL of triethylamine (TCI CO., LTD.) were mixed with this solution and the mixture was stirred at 50°C for 24 hours. Then, the mixture was diluted with 1.0 mL of ion-exchanged water, and placed in a cellulose dialysis tube (seamless cellulose tube, Spectrum). Both ends of the tube were tied, and then it was dialyzed with ion-exchanged water for 5 days. After purification, the solution in the tube was freeze-dried, and 11.5 mg of polymer compound in which mono-L-arginine was introduced (hereinafter sometimes referred to as NVA-AA-R1/L), was obtained. According to GPC measurement, the molecular weight was 375000 (PEG/PEO conversion).

### (Measurement of guanidino group)

For the obtained polymer compound, the content of groups originating from arginine was determined by measuring ¹H-NMR, and the density of guanidine groups per polymer compound was calculated from this result.
¹H-NMR(400MHz, D₂O):δ=4.16-3.75(br, 1H), 3.50-3.31(br, 0.67H)

Specifically, the procedure was as follows. First, ¹H-NMR of the NVA-AA polymer before the introduction of arginine is measured, and to thereby calculate the x/(y + z) ratio of the "repetition unit" of the following structural formula (4).

Next, x/z is calculated from a value of integral of one hydrogen atom of (*1) and two hydrogen atoms of (*2) in the following formula (4). From these results, the x/y/z ratio is obtained. (14/1.32/4.68 in Example 1)

The term "repetition unit" used herein means a repeating structure based on the composition ratio of monomer units (in Example 1, the portion derived from N-vinylacetamide, the portion derived from acrylic acid, and the portion derived from acrylic acid to which a peptide chain is bonded). When the main chain is a random copolymer, for convenience, the "repetition unit" is determined assuming that the block arrangements based on the composition ratio are repeated.

The mole number of guanidine groups contained in one repeating unit (x/y/z = 14/1.32/4.68 in Example 1) can be calculated by multiplying the number of repeating arginine contained in the basic peptide by z. That is, the mole number of the guanidine group contained in the unit structure can be calculated. (In this example, arginine is one, and thus the number of repeating arginine is one).

The density of guanidino groups in the polymer compound was calculated by dividing the obtained "mole number of guanidine groups" by the molecular weight (weight for convenience) of the unit structure having the above x/y/z ratio. The results are shown in Table 1.

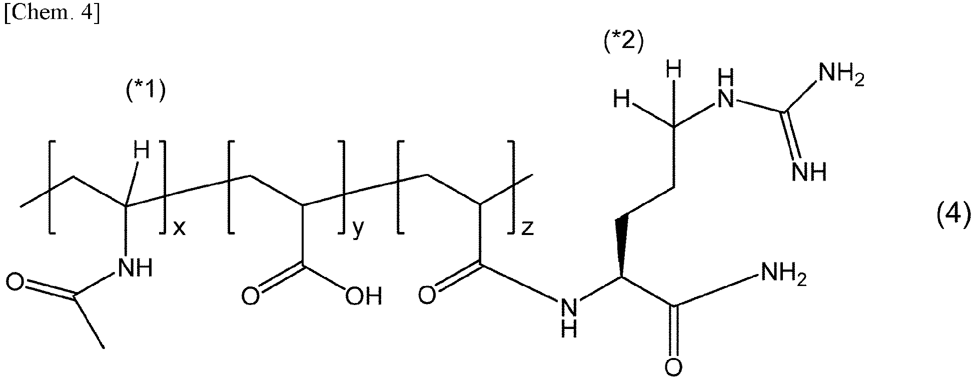

### «Example 2» (not according to the invention)

In this example, acrylic acid-N-vinylacetamide copolymer was used as a main chain polymer, to prepare a polymer compound having two arginine in side chains.

The procedure described in Example 1 was repeated, except that a basic peptide consisting of two molecules of L-arginine (NH₂-R2/L) was used instead of the mono-L-arginine, to obtain 16.5mg of the polymer compound wherein two arginine were introduced (hereinafter sometimes referred to as NVA-AA-R2/L). According to GPC measurement, the molecular weight was 482000 (PEG/PEO conversion). The density of guanidine groups was calculated in the same manner as in Example 1. The results are shown in Table 1.

### «Example 3»

In this example, acrylic acid-N-vinylacetamide copolymer was used as a main chain polymer, to prepare a polymer compound having four arginine in side chains.

The procedure described in Example 1 was repeated, except that a basic peptide consisting of four molecules of L-arginine (NH₂-R4/L) was used instead of the mono-L-arginine, to obtain 20.3mg of the polymer compound wherein four arginine were introduced (hereinafter sometimes referred to as NVA-AA-R4/L). According to GPC measurement, the molecular weight was 531000 (PEG/PEO conversion). The density of guanidine groups was calculated in the same manner as in Example 1. The results are shown in Table 1.

### «Comparative Example 1»

In this comparative example, acrylic acid-N-vinylacetamide copolymer was used as a main chain polymer, to prepare a polymer compound having eight arginine in side chains.

The procedure described in Example 1 was repeated, except that a basic peptide consisting of eight molecules of L-arginine (NH₂-R8/L) was used instead of the mono-L-arginine, to obtain 17.8mg of the polymer compound wherein eight arginine were introduced (hereinafter sometimes referred to as NVA-AA-R8/L). According to GPC measurement, the molecular weight was 671000 (PEG/PEO conversion). The density of guanidine groups was calculated in the same manner as in Example 1. The results are shown in Table 1.

### «Comparative Example 2»

In this comparative example, acrylic acid-N-vinylacetamide copolymer was used as a main chain polymer, to prepare a polymer compound having eight arginine in side chains.

The procedure described in Example 1 was repeated, except that a basic peptide consisting of eight molecules of D-arginine was used instead of the mono-L-arginine, to obtain 17.8mg of the polymer compound wherein eight D-arginine were introduced (hereinafter sometimes referred to as NVA-AA-R8/D). According to GPC measurement, the molecular weight was 611000 (PEG/PEO conversion). The density of guanidine groups was calculated in the same manner as in Example 1. The results are shown in Table 1.

### «Measurement of uptake into cells»

The uptake of FITC-OVA into cells was measured using the polymer compounds obtained in Examples 1 to 3 (Examples 1 and 2 not according to the invention) and Comparative Examples 1 to 2.

500 µL of a suspension of Chinese hamster ovary cells (CHO cells; 2 × 10⁵ cells/mL) in a Ham's F12 medium was seeded to each well of a 24-well plate, and precultured for 24 hours in a CO₂ incubator (37 ° C, 5% CO₂). After removing the supernatant and washing twice with 500 µL of phosphate-buffered physiological saline, 250µL of a solution of fluorescein-labeled ovoalbumin (FITC-OVA; Thermo Fisher Scientific) in Ham's F12 medium was added (final concentration of 5µg/mL). Further, 250µL of a solution of each of polymer compounds obtained in Examples 1 to 3 and Comparative Examples 1 to 2 in Ham's F12 medium was added thereto (final concentration of 1µg/mL), and cultured for 2 hours in a CO₂ incubator.
The supernatant of the culture medium was removed, and the cells were washed twice with 500µL of phosphate buffered physiological saline, and then 100µL of trypsin EDTA solution (Life Technologies) was added, and the cultured CHO cells were separated from the plate and dispersed. Next, 400µL of solution of trypan blue was added thereto, and the cells were suspended and then collected in a microtube. The collected cell suspension was passed through a cell strainer and MFI (mean fluorescence intensity) was measured using flow cytometry. As a control, only the acrylic acid-N-vinylacetamide copolymer (main chain polymer) was added, only the basic peptide consisting of four arginine (R4/L) was added, only FITC-OVA (FITC-OVA) was added, and only cells (Cells) were added. The results are shown in Table 1.

Extracellular FITC-OVA is quenched by trypan blue and thus emits no fluorescence, and only FITC-OVA that has introduced into a cell emits fluorescence. MFI refers to a mean value of fluorescence intensity per cell, and therefore, a larger MFI value means that FITC-OVA, which is a water-soluble polymer compound was taken up by a cell in a larger amount.

In addition, the "uptake efficiency" of FITC-OVA was calculated by flow cytometric analysis. In the "uptake efficiency", the maximum value of autofluorescence intensity when only cells are measured is used as a reference value. The "uptake efficiency" is the ratio of the number of cells that shows a value higher than the above reference value when FITC-OVA is taken up by cells.

The results are shown in Table 1.

### «Measurement of cytotoxicity»

Cytotoxicity was measured using Cytotoxicity LDH Assay Kit-WST(Dojindo Molecular Technologies, Inc).

100 µL of a suspension of CHO cells (2 × 10⁵ cells/mL) in a Ham's F12 medium was seeded to each well of a 96-well plate, and precultured for 24 hours in a CO₂ incubator (37 ° C, 5% CO₂). 220µL of a solution of each of polymer compounds obtained in Examples 1 to 3 and Comparative Examples 1 to 2 in Ham's F12 medium was added thereto (final concentration of 5µg/mL). Further, 220µL of Ham's F12 medium was added to a well for low control, 200µL of Ham's F12 medium was added to a well for high control, and 220µL of Ham's F12 medium was added to a well for background (without cells). Then, they were cultured for 1.5 hours in the CO₂ incubator. Then, 20µL of Lysis Buffer was added to the well for high control, and they were cultured for 30 minutes in the CO₂ incubator. 100µL of supernatant of each well was collected, and transferred to a 96-well plate for measurement. 100µL of Working Solution was added to all wells, and a color reaction was carried out for 30 minutes at room temperature under light shielding. Then, 50 µL of Stop Solution was added to all wells. Absorbance at 490 nm was measured using a plate reader, and cytotoxicity (%) was calculated. The results are shown in Table 1.

**[Table 1] (Examples 1 and 2 not according to the invention)**

| | Main chain polymer | Side chain | Content of guanidino group (mmol/g) | MFI | Uptake efficiency (%) | Cytotoxicity (%) |
|---|---|---|---|---|---|---|
| Example 1 | NVA-AA | R1/L | 2.0 | 30.13 | 76 | 1.5 |
| Example 2 | NVA-AA | R2/L | 3.3 | 134.80 | 94 | 3.1 |
| Example 3 | NVA-AA | R4/L | 4.4 | 166.70 | 96 | 6.9 |
| Comparative Example 1 | NVA-AA | R8/L | 4.4 | 176.90 | 95 | 8.7 |
| Comparative Example 2 | NVA-AA | R8/D | 4.6 | 258.99 | 97 | 8.9 |
| Main chain polvmer | NVA-AA | - | - | 4.32 | - | - |
| R4/L | - | R4/L | - | 4.36 | - | - |
| FITC-OVA | - | - | - | 4.34 | - | - |
| Cells | - | - | - | 4.04 | - | - |

| | | | | | | |
|---|---|---|---|---|---|---|
| *Introduced protein: FITC-OVA(1µg/mL) *Concentration of added polymer compound of Examples 1 to 3 and Comparative Examples 1 and 2, main chain polymer R4/L, at measurement: 5µg/mL | | | | | | |

When the polymer compounds obtained in Examples 1 to 3 (Examples 1 and 2 not according to the invention) were used, higher MFIs of 30.13 to 166.70 were shown as compared with MFIs of 4.04 to 4.36 of the controls. In addition, regarding the uptake efficiency into cells, the polymer compounds of Examples 2 and 3 (Example 2 not according to the invention) showed almost the same uptake efficiency as that of Comparative Example 1. The polymer compound of Example 1 (not according to the invention) was also taken up by 76% of cells. On the other hand, regarding the cytotoxicity, the polymer compounds of Examples 1 to 3 (Examples 1 and 2 not according to the invention) showed low cytotoxicity of 1.5% to 6.9% as compared with 8.7% and 8.9% of Comparative Examples 1-1 and 1-2.

### «Example 4» (not according to the invention)

In this example, hyaluronic acid (molecular weight:30,000) was used as a main chain polymer, to prepare a polymer compound having two glycine and two arginine in side chains.

20.0mg of hyaluronic acid (TCI, average molecular weight:30,000) was dissolved in 0.8mL of dimethylsulfoxide (DMSO) at 60°C by stirring. To this solution, 18mg of N-hydroxysuccinimide dissolved in 0.15mL of DMSO was added, and further 34.0mg of dicyclohexylcarbodiimide (DCC) dissolved in 0.15mL of DMSO was added thereto, and the mixture was stirred for 24 hours. A solid precipitate was filtered out by filtration to obtain a solution of a succinimide ester of hyaluronic acid in DMSO. 0.40 mL of a solution (116mg/mL) of a basic peptide (NH₂-G2R2/L, SIGMA) constituting of two glycine and two L-arginine in DMSO and 0.04 mL of triethylamine (TCI CO., LTD.) were mixed into the solution of the succinimide ester of hyaluronic acid in DMSO, and the resulting solution was stirred at room temperature for 24 hours. After the reaction, the reaction solution was diluted with 1.0 mL of ion-exchanged water, and placed in a cellulose dialysis tube (seamless cellulose tube, Spectrum). Both ends of the tube were tied, and then it was dialyzed with ion-exchanged water for 5 days. Then, the solution in the tube was freeze-dried, and 14.0 mg of polymer compound in which two arginine were introduced (hereinafter sometimes referred to as HA(30k)-G2R2/L), was obtained.

### (Measurement of guanidino group)

For the obtained polymer compound (HA-G2R2/L), the content of groups originating from arginine was determined by measuring ¹H-NMR, and the density of guanidine groups per polymer compound was calculated from this result.
¹H-NMR(400MHz, D₂O):δ=3.45-3.34(br, 1.64H), 2.26-2.12(br, 3H)

Specifically, the procedure was as follows.

First, l/m ratio is calculated from a value of integral of three hydrogen atoms of (*3) and two hydrogen atoms of (*4) in the following formula (5). (0.41/0.59 in Example 4)

The mole number of guanidine groups contained in one repeating unit (l/m=0.41/0.59 in Example 4) can be calculated by multiplying the number of repeating arginine contained in the basic peptide by l. That is, the mole number of the guanidine group contained in the unit structure can be calculated. (In this example 4, arginine is two, and thus the number of repeating arginine is two).

The density of guanidino groups in the polymer compound was calculated by dividing the obtained "mole number of guanidine groups" by the molecular weight (weight for convenience) of the unit structure having the above l/m ratio. The results are shown in Table 2.

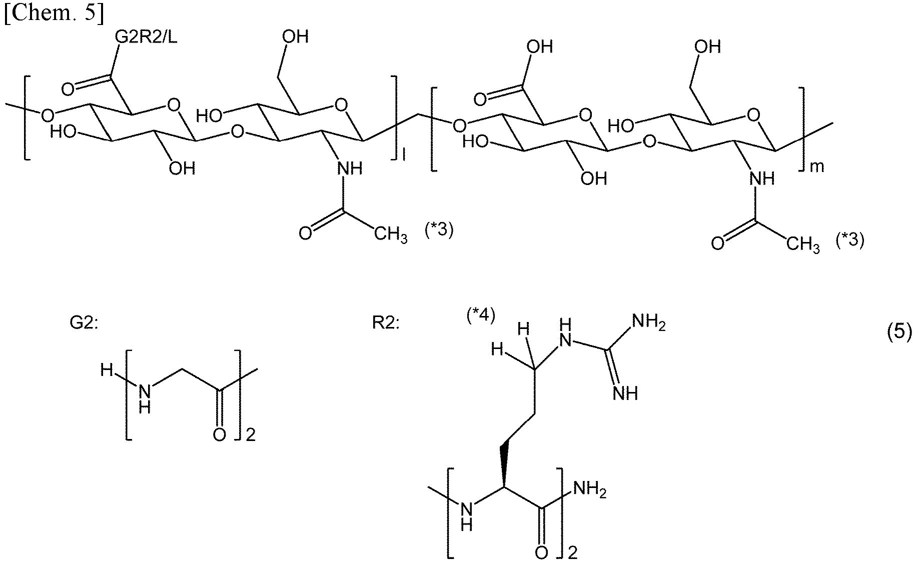

### «Example 5» (not according to the invention)

In this example, hyaluronic acid (molecular weight:220,000) was used as a main chain polymer, to prepare a polymer compound having two glycine and two arginine in side chains.

The procedure described in Example 4 was repeated, except that hyaluronic acid with 220,000 of molecular weight was used instead of hyaluronic acid with 30,000 of molecular weight, to obtain 21.0mg of the polymer compound wherein two glycine and two arginine were introduced (hereinafter sometimes referred to as HA(220k)-G2R2).

In this example, two types of HA (220k) -G2R2/L with different densities of guanidine group were obtained. In Table 2, they are described as Example 5-1 and Example 5-2, respectively. The density was calculated in the same manner as in Example 4. The results are shown in Table 2.

### «Measurement of uptake into cells»

The uptake of FITC-BSA into cells was measured using the polymer compounds obtained in Examples 4 to 5. The procedure of measurement of uptake into cells in Examples 1 to 3, and Comparative Examples 1 to 2 was repeated, except that a fluorescein-labelled bovine serum albumin (FITC-BSA; Thermo Fisher Scientific) was used instead of the FITC-OVA, and the polymer compounds obtained in Examples 4 to 5, the main chain polymer, the added concentration of G2R2, and the concentration of FITC-BSA were changed. The results are shown in Table 2.

**[Table 2] (Examples 4, 5-1 and 5-2 not according to the invention)**

| | Main chain polymer | Side chain | Content of guanidino group (mmol/g) | MFI | Uptake efficiency (%) |
|---|---|---|---|---|---|
| Example 4 | HA(30k) | G2R2/L | 1.5 | 42.71 | 94 |
| Example 5-1 | HA(220k) | G2R2/L | 1.6 | 268.24 | 99 |
| Example 5-2 | HA(220k) | G2R2/L | 2.4 | 1761.48 | 99 |
| Main chain polymer | HA(30k) | - | - | 5.02 | - |
| G2R2/L | - | G2R2/L | - | 4.26 | - |
| FITC-BSA | - | - | - | 5.05 | - |
| Cells | - | - | - | 3.56 | - |

| | | | | | |
|---|---|---|---|---|---|
| *Introduced protein: FITC-BSA(10µg/mL) *Concentration of added polymer compound of Examples 4 and 5, main chain polymer G2R2, at measurement: 50µg/mL | | | | | |

When the polymer compounds obtained in Examples 4 to 5 (not according to the invention) were used, higher MFIs of 42.71 to 1761.48 were shown as compared with MFIs of 4.26 to 5.05 of the controls.

### «Example 6» (not according to the invention)

In this example, γ-Polyglutamic acid (hereinafter referred to as γ-PGA) was used as a main chain polymer, to prepare a polymer compound having one arginine in side chains.

### (Synthesis of succinimide of γ-PGA)

10mg of γ-PGA(Wako Pure Chemical Corporation, average molecular weight:200,000 to 500,000) and 1.0mL of DMSO were applied, and dissolved at 70°C by stirring, and then cooled to room temperature. To this solution, 248.8mg of DMSO solution containing N, N'- dicyclohexylcarbodiimide (0.12g/mL) and 236.3mg of DMSO solution containing N-hydroxysuccinimide (0.067g/mL) was added, and the mixture was stirred for 24 hours at room temperature. Subsequently, a filtrate was recovered by suction filtration of the reaction solution, to obtain the DMSO solution of the succinimide of γ-PGA (γ-PGA-OSu).

### (Introduction of basic peptide)

428.9mg of DMSO solution containing mono-L-arginine (NH2-R1/L, Merck Corporation) (72.3mg/mL) and 0.02mL of triethylamine (TCI CO., LTD.) were mixed with the DMSO solution of γ-PGA-OSu, and the mixture was stirred at room temperature for 24 hours.
Then, the mixture was diluted with 2.0 mL of ion-exchanged water, and placed in a cellulose dialysis tube (seamless cellulose tube, Spectrum). Both ends of the tube were tied, and then it was dialyzed with ion-exchanged water for 5 days. After purification, the solution in the tube was freeze-dried, and 8.8 mg of polymer compound in which di-L-arginine was introduced (hereinafter sometimes referred to as γ-PGA-R2/L), was obtained. According to GPC measurement, the molecular weight was 7,180 (PEG/PEO conversion).

### (Measurement of guanidino group)

For the obtained polymer compound(γ-PGA-R1/L), the content of groups originating from arginine was determined by measuring ¹H-NMR, and the density of guanidine groups per polymer compound was calculated from this result.
¹H-NMR(400MHz, D₂O):δ=3.20-3.05(br, 0.67H), 2.49-2.19(br, 1H)

Specifically, the procedure was as follows.

First, p/q ratio is calculated from a value of integral of two hydrogen atoms of (*5) and two hydrogen atoms of (*6) in the following formula (6). (0.33/0.67 in Example 6)

The mole number of guanidine groups contained in one repeating unit (p/q=0.33/0.67 in Example 6) can be calculated by multiplying the number of repeating arginine contained in the basic peptide by q. That is, the mole number of the guanidine group contained in the unit structure can be calculated. (In this example 6, arginine is one, and thus the number of repeating arginine is one).

The density of guanidino groups in the polymer compound was calculated by dividing the obtained "mole number of guanidine groups" by the molecular weight (weight for convenience) of the unit structure having the above p/q ratio. The results are shown in Table 3.

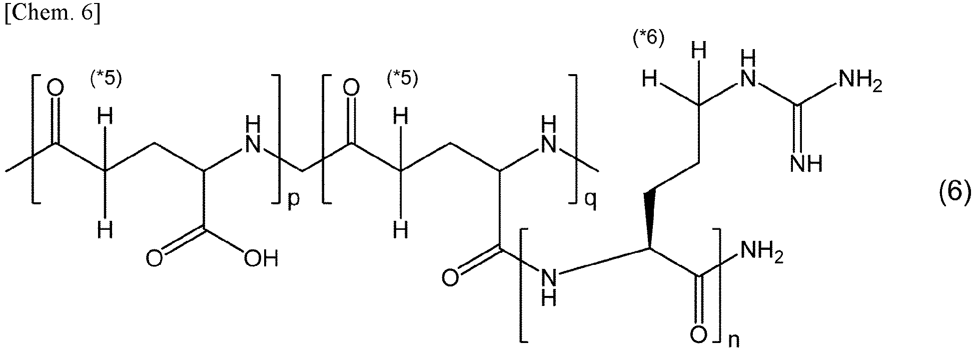

### «Example 7» (not according to the invention)

In this example, γ-PGA was used as a main chain polymer, to prepare a polymer compound having two arginine in side chains.

The procedure described in Example 6 was repeated, except that a basic peptide consisting of two molecules of L-arginine (NH₂-R2/L) was used instead of the mono-L-arginine, to obtain 20.2mg of the polymer compound wherein two arginine were introduced (hereinafter sometimes referred to as γ-PGA-R2/L). According to GPC measurement, the molecular weight was 13,900 (PEG/PEO conversion). The density of guanidine groups was calculated in the same manner as in Example 6. The results are shown in Table 3.

### «Example 8»

In this example, γ-PGA was used as a main chain polymer, to prepare a polymer compound having four arginine in side chains.

The procedure described in Example 6 (not according to the invention) was repeated, except that a basic peptide consisting of four molecules of L-arginine (NH₂-R4/L) was used instead of the mono-L-arginine, to obtain 21.1mg of the polymer compound wherein four arginine were introduced (hereinafter sometimes referred to as γ-PGA-R4/L). According to GPC measurement, the molecular weight was 7,630 (PEG/PEO conversion). The density of guanidine groups was calculated in the same manner as in Example 6. The results are shown in Table 3.

### «Example 9» (not according to the invention)

In this example, γ-PGA was used as a main chain polymer, to prepare a polymer compound having one glycine and two arginine in side chains.

The procedure described in Example 6 (not according to the invention) was repeated, except that a basic peptide consisting of one glycine and two arginine (NH₂-G1R2/L) was used instead of the mono-L-arginine, to obtain 29.3mg of the polymer compound wherein four arginine were introduced (hereinafter sometimes referred to as γ-PGA-G1R2/L). According to GPC measurement, the molecular weight was 14,000 (PEG/PEO conversion). The density of guanidine groups was calculated in the same manner as in Example 6. The results are shown in Table 3.

### «Measurement of uptake into cells»

The uptake of FITC-OVA into cells was measured using the polymer compounds obtained in Examples 6 to 10 (Examples 6, 7, 9 and 10 not according to the invention).

500 µL of a suspension of Chinese hamster ovary cells (CHO cells; 2 × 10⁵ cells/mL) in a Ham's F12 medium was seeded to each well of a 24-well plate, and precultured for 24 hours in a CO₂ incubator (37 ° C, 5% CO₂). After removing the supernatant and washing twice with 500 µL of phosphate-buffered physiological saline, 250µL of a solution of fluorescein-labeled ovoalbumin (FITC-OVA; Thermo Fisher Scientific) in Ham's F12 medium was added (final concentration of 1µg/mL). Further, 250µL of a solution of each of polymer compounds obtained in Examples 6 to 10 (Examples 6, 7, 9 and 10 not according to the invention) in Ham's F12 medium was added thereto (final concentration of 5µg/mL), and cultured for 2 hours in a CO₂ incubator. The supernatant of the culture medium was removed, and the cells were washed twice with 500µL of phosphate buffered physiological saline, and then 100µL of trypsin EDTA solution (Life Technologies) was added, and the cultured CHO cells were separated from the plate and dispersed. Next, 400µL of solution of trypan blue was added thereto, and the cells were suspended and then collected in a microtube. The collected cell suspension was passed through a cell strainer and MFI (mean fluorescence intensity) was measured using flow cytometry. As a control, only γ-PGA (main chain polymer) was added, only the basic peptide consisting of four arginine (R4/L) was added, only FITC-OVA (FITC-OVA) was added, and only cells (Cells) were added. The results are shown in Table 3.

Extracellular FITC-OVA is quenched by trypan blue and thus emits no fluorescence, and only FITC-OVA that has introduced into a cell emits fluorescence. MFI refers to a mean value of fluorescence intensity per cell, and therefore, a larger MFI value means that FITC-OVA, which is a water-soluble polymer compound was taken up by a cell in a larger amount.

In addition, the "uptake efficiency" of FITC-OVA was calculated by flow cytometric analysis. In the "uptake efficiency", the maximum value of autofluorescence intensity when only cells are measured is used as a reference value. The "uptake efficiency" is the ratio of the number of cells that shows a value higher than the above reference value when FITC-OVA is taken up by cells.

The results are shown in Table 3.

### «Measurement of cytotoxicity»

A cytotoxicity was measured using Cytotoxicity LDH Assay Kit-WST(Dojindo Molecular Technologies, Inc).

100 µL of a suspension of CHO cells (2 × 10⁵ cells/mL) in a Ham's F12 medium was seeded to each well of a 96-well plate, and precultured for 24 hours in a CO₂ incubator (37 ° C, 5% CO₂). 220µL of a solution of each of polymer compounds obtained in Examples 6 to 10 in Ham's F12 medium was added thereto (final concentration of 5µg/mL). Further, 220µL of Ham's F12 medium was added to a well for low control, 200µL of Ham's F12 medium was added to a well for high control, and 220µL of Ham's F12 medium was added to a well for background (without cells). Then, they were cultured for 1.5 hours in the CO₂ incubator. Then, 20µL of Lysis Buffer was added to the well for high control, and they were cultured for 30 minutes in the CO₂ incubator. 100µL of supernatant of each well was collected, and transferred to a 96-well plate for measurement. 100µL of Working Solution was added to all wells, and a color reaction was carried out for 30 minutes at room temperature under light shielding. Then, 50 µL of Stop Solution was added to all wells. Absorbance at 490 nm was measured using a plate reader, and cytotoxicity (%) was calculated. The results are shown in Table 3.

**[Table 3] (Examples 6, 7, and 9 not according to the invention)**

| | Main chain polymer | Side chain | Content of guanidino group (mmol/g) | MFI | Uptake efficiency (%) | Cytotoxicity (%) |
|---|---|---|---|---|---|---|
| Example 6 | γ-PGA | R1/L | 2.8 | 13 | 64 | n.d. |
| Example 7 | γ-PGA | R2/L | 3.8 | 36 | 88 | n.d. |
| Example 8 | γ-PGA | R4/L | 4.2 | 14 | 67 | n.d. |
| Example 9 | γ-PGA | G1R2/L | 3.7 | 24 | 82 | n.d. |
| Main chain polymer | γ-PGA | - | - | - | - | - |
| R4/L | - | R4/L | - | 4.4 | - | - |
| FITC-OVA | - | - | - | 3.2 | - | - |
| Cells | - | - | - | 3.2 | - | - |

| | | | | | | |
|---|---|---|---|---|---|---|
| *Introduced protein: FITC-OVA(1µg/mL) *Since γ-PGA is insoluble in water, it is not possible to measure uptake into cells and to measure cytotoxicity. *Concentration of added polymer compound of Examples 6 to 9, main chain polymer, R4/L, at measurement: 5µg/mL | | | | | | |

When the polymer compounds obtained in Examples 6 to 9 (Examples 6, 7 and 9 not according to the invention) were used, higher MFIs of 13 to 36 were shown as compared with MFIs of 3.2 to 4.4 of the controls.

### INDUSTRIAL APPLICABILITY

The polymer compound of the present invention may be used for introducing the low membrane permeable drug into cells.

## Claims

1. A polymer compound having a weight average molecular weight of 1000 or more, wherein the polymer compound has, at an end of a side chain, a basic peptide of chain length of 3 to 6 containing three or more arginine, and comprises a guanidino group density of 0.5 to 20 mmol/g, originating from arginine,
wherein the weight average molecular weight is obtained by GPC analysis using an aqueous solvent, and
wherein the guanidino group density is determined by measuring a hydrogen atom derived from the main chain of the polymer and a hydrogen atom derived from arginine by ¹H-NMR, respectively, and calculating the amount of the guanidino group derived from arginine.

2. The polymer compound according to claim 1, wherein a main chain of the polymer compound is one or a combination of two or more selected from the group consisting of a hydrophilic polymer, an anionic polymer, and a polysaccharide derivative.

3. The polymer compound according to claim 1 or 2, wherein an end of the basic peptide is -CONH₂ group.

4. The polymer compound according to any one of claims 1 to 3, wherein the basic peptide is consisting of arginine and glycine.

5. An agent for promoting introduction of compound into cells, comprising the polymer compound according to any one of claims 1 to 4.

6. A pharmaceutical composition, comprising the polymer compound according to any one of claims 1 to 4, and a drug.

## Patentansprüche

1. Polymerverbindung mit einem gewichtsmittleren Molekulargewicht von 1000 oder mehr, wobei die Polymerverbindung an einem Ende einer Seitenkette ein basisches Peptid mit einer Kettenlänge von 3 bis 6 aufweist, das drei oder mehr Arginin enthält, und eine Guanidinogruppen-Dichte von 0,5 bis 20 mmol/g aufweist, die aus Arginin stammt,
wobei das gewichtsmittlere Molekulargewicht durch GPC-Analyse unter Verwendung eines wässrigen Lösungsmittels ermittelt wird, und
wobei die Guanidinogruppen-Dichte bestimmt wird, indem ein aus der Hauptkette des Polymers stammendes Wasserstoffatom und ein aus Arginin stammendes Wasserstoffatom jeweils mittels ¹H-NMR gemessen werden und die Menge der aus Arginin stammenden Guanidinogruppen berechnet wird.

2. Die Polymerverbindung nach Anspruch 1, wobei eine Hauptkette der Polymerverbindung eine oder eine Kombination aus zwei oder mehr ist, ausgewählt aus der Gruppe bestehend aus einem hydrophilen Polymer, einem anionischen Polymer und einem Polysaccharidderivat.

3. Die Polymerverbindung nach Anspruch 1 oder 2, wobei ein Ende des basischen Peptids eine -CONH₂-Gruppe ist.

4. Die Polymerverbindung gemäß einem der Ansprüche 1 bis 3, wobei das basische Peptid aus Arginin und Glycin besteht.

5. Ein Mittel, das das Eindringen einer Verbindung in Zellen fördert, umfassend die Polymerverbindung gemäß einem der Ansprüche 1 bis 4.

6. Eine pharmazeutische Zusammensetzung, die die Polymerverbindung gemäß einem der Ansprüche 1 bis 4 und einen Wirkstoff umfasst.

## Revendications

1. Composé polymère ayant un poids moléculaire moyen en poids de 1 000 ou plus, dans lequel le composé polymère comporte, à une extrémité d'une chaîne latérale, un peptide basique d'une longueur de chaîne de 3 à 6 contenant trois arginines ou plus, et comprend une densité de groupes guanidino de 0,5 à 20 mmol/g, provenant de l'arginine,
dans lequel le poids moléculaire moyen en poids est obtenu par analyse GPC utilisant un solvant aqueux, et
dans lequel la densité en groupes guanidino est déterminée en mesurant respectivement un atome d'hydrogène dérivé de la chaîne principale du polymère et un atome d'hydrogène dérivé de l'arginine par ¹RMN H, et en calculant la quantité du groupe guanidino dérivé de l'arginine.

2. Composé polymère selon la revendication 1, dans lequel une chaîne principale du composé polymère est un ou une combinaison de deux ou plusieurs éléments choisis dans le groupe constitué d'un polymère hydrophile, d'un polymère anionique et d'un dérivé de polysaccharide.

3. Composé polymère selon la revendication 1 ou 2, dans lequel une extrémité du peptide basique est un groupe -CONH₂.

4. Composé polymère selon l'une quelconque des revendications 1 à 3, dans lequel le peptide basique est constitué d'arginine et de glycine.

5. Agent destiné à favoriser l'introduction d'un composé dans les cellules, comprenant le composé polymère selon l'une quelconque des revendications 1 à 4.

6. Composition pharmaceutique, comprenant le composé polymère selon l'une quelconque des revendications 1 à 4, et un médicament.
